# EUROPEAN PATENT APPLICATION

(11) **EP 3 674 416 A1**
(43) Date of publication of application: **01.07.2020**
(21) Application number: 18860871.5
(22) Date of filing: 27.09.2018
(51) Int. Cl.: C12Q 1/6813, C12N 15/09, C12Q 1/6886, G01N 33/53

(54) **METHOD AND KIT FOR PREDICTING THERAPEUTIC EFFECTIVENESS OF CHEMOTHERAPY FOR DIFFUSE LARGE B-CELL LYMPHOMA PATIENTS**

(30) Priority: 29.09.2017 US 201762565123 P
(71) Applicant: Kyushu University, National University Corporation, Fukuoka-shi, Fukuoka 819-0395 (JP)
(72) Inventor: MIYAWAKI, Kohta, Fukuoka-shi Fukuoka 8190395 (JP); AKASHI, Koichi, Fukuoka-shi Fukuoka 8190395 (JP); MAEDA, Takahiro, Fukuoka-shi Fukuoka 8190395 (JP); KATO, Koji, Fukuoka-shi Fukuoka 8190395 (JP)
(74) Representative: EP&C
(86) International application number: PCT/JP2018/036080
(87) International publication number: WO 2019/065901

(57) **Abstract**

A method is provided for predicting the therapeutic effectiveness of chemotherapy for a diffuse large B-cell lymphoma patient, including: at least one step selected from the group consisting of a step of measuring expression of a marker of a T cell in a sample obtained from the patient, a step of measuring expression of a marker of a macrophage or a dendritic cell in a sample obtained from the patient, and a step of measuring expression of a marker of a stromal cell in a sample obtained from the patient; and a step of predicting prognosis of the treatment, based on the expression of the marker.

## Description

### TECHNICAL FIELD

The present invention relates to a method and a kit for predicting therapeutic effectiveness of chemotherapy in diffuse large B-cell lymphoma patients.

Priority is claimed on U.S. Provisional Patent Application No. 62/565,123 filed on September 29, 2017, the content of which is incorporated herein by reference.

### BACKGROUND ART

The response of diffuse large B-cell lymphoma (hereinafter, also referred to as DLBCL), which is the largest type and accounts for over 30% of all lymphomas, to R-CHOP therapy, which is standard therapy, is diverse. Therefore, the establishment of a stratification system that accurately predicts cases with favorable prognosis or treatment resistance at the first onset is critical. Currently, as a prognostic stratification method, classification based on a differentiation stage (cell-of-origin; COO) of tumor B cells (hereinafter, also referred to as COO classification) is mainly used (Non-Patent Document 1).

However, in recent large-scale clinical trial results, stratification based on COO classification did not necessarily reflect prognosis (For example, Non-Patent Document 2).

### Citation List

### Non-Patent Document

[Non-Patent Document 1] Nature 403, 503-511 (2000)
[Non-Patent Document 2] J. Clin. Oncol. 35(22), 2515-2526 (2017)

### DISCLOSURE OF INVENTION

### Technical Problem

In the prognostic stratification of R-CHOP therapy, stratification based on a COO classification of tumor B cells of the related art does not always reflect the prognosis. Therefore, the stratification using the COO classification has a problem in that the therapeutic effectiveness of chemotherapy such as R-CHOP for DLBCL patients cannot be predicted.

An object of the present invention is to provide a method and a kit for predicting therapeutic effectiveness of chemotherapy on a DLBC patient, based on lymphoma microenvironment signature.

### Solution to Problem

The present inventors have found that therapeutic effectiveness of chemotherapy on diffuse large B-cell lymphoma patients can be stratified by expressing a gene derived from microenvironment cells of the diffuse large B-cell lymphoma and an effect of the chemotherapy can be predicted, and have completed the present invention.

The present invention has the following aspects.
[1] A method for predicting therapeutic effectiveness of chemotherapy for a diffuse large B-cell lymphoma patient, including:
   at least one step selected from the group consisting of a step of measuring expression of a marker of a T cell in a sample obtained from the patient, a step of measuring expression of a marker of a macrophage or a dendritic cell in a sample obtained from the patient, and a step of measuring expression of a marker of a stromal cell in a sample obtained from the patient; and
   a step of predicting prognosis of the treatment, based on the expression of the marker.
[2] The method according to [1], in which the step of measuring the expression of the marker is a step of measuring an mRNA expression level of the marker.
[3] The method according to [2], in which the step of predicting the prognosis of the treatment based on the expression of the marker is a step of indicating that the prognosis of the treatment is favorable, when the mRNA expression level of the marker is equal to or more than a cutoff value determined by ROC analysis.
[4] The method according to any one of [1] to [3], in which the chemotherapy is chemotherapy in combination with anti-CD20 antibody.
[5] The method according to any one of [1] to [4], in which the marker of the T cell is at least one marker selected from the group consisting of CCR4, CD2, CD3D, CD3E, CD6, CD7, CD96, FAS, IL2, IL2RB, IL2RG, IL7R, ITK, ITPKB, TNFRSF9, TRAF1, CD40LG, CTLA4, CXCL13, ICOS, IL21, PDCD1, SH2D1A, SLAMF1, and TNFRSF4.
[6] The method according to any one of [1] to [5], in which the T cell is a follicular helper T cell (Tfh cell).
[7] The method according to [6], in which a marker of the Tfh cell is at least one marker selected from the group consisting of CD40LG, CTLA4, CXCL13, ICOS, IL21, PDCD1, SH2D1A, SLAMF 1, and TNFRSF4.
[8] The method according to any one of [1] to [7], in which the marker of the macrophage or the dendritic cell is at least one marker selected from the group consisting of CD80, HLA-DRB1, CD11c (ITGAX), and NOD2.
[9] The method according to any one of [1] to [8], in which the marker of the stromal cell is at least one marker selected from the group consisting of BMP2K, COL8A2, FGFR1, and OBSCN.
[10] A kit for predicting therapeutic effectiveness of chemotherapy for a diffuse large B-cell lymphoma patient, including:
   at least one reagent selected from the group consisting of a reagent for measuring expression of a marker of a T cell, a reagent for measuring expression of a marker of a macrophage or a dendritic cell, and a reagent for measuring expression of a marker of a stromal cell.
[11] The kit according to [10], in which the reagent for measuring the expression of the marker is a reagent for measuring an mRNA expression level of the marker.
[12] The kit according to [10] or [11], in which the chemotherapy is chemotherapy in combination with anti-CD20 antibody.
[13] The kit according to any one of [10] to [12], in which the marker of the T cell is at least one marker selected from the group consisting of CCR4, CD2, CD3D, CD3E, CD6, CD7, CD96, FAS, IL2, IL2RB, IL2RG, IL7R, ITK, ITPKB, TNFRSF9, TRAF1, CD40LG, CTLA4, CXCL13, ICOS, IL21, PDCD1, SH2D1A, SLAMF1, and TNFRSF4.
[14] The kit according to any one of [10] to [13], in which the T cell is a follicular helper T cell (Tfh cell).
[15] The kit according to [14], in which a marker of the Tfh cell is at least one marker selected from the group consisting of CD40LG, CTLA4, CXCL13, ICOS, IL21, PDCD1, SH2D1A, SLAMF 1, and TNFRSF4.
[16] The kit according to any one of [10] to [15], in which the marker of the macrophage or the dendritic cell is at least one marker selected from the group consisting of CD80, HLA-DRB1, CD11c (ITGAX), and NOD2.
[17] The kit according to any one of [10] to [16], in which the marker of the stromal cell is at least one marker selected from the group consisting of BMP2K, COL8A2, FGFR1, and OBSCN.

### Advantageous Effects of Invention

According to the present invention, it is possible to provide a method and a kit for predicting therapeutic effectiveness of chemotherapy for DLBCL patients.

### BRIEF DESCRIPTION OF DRAWINGS

FIG. 1A is a diagram obtained by analyzing expression levels of 48 representative genes in FFPE samples from DLBCL patients, using nCounter (registered trademark) system.
FIG. 1B is a diagram showing survival curves of 15 DLBCL patients of each of favorable prognosis and poor prognosis, after R-CHOP therapy.
FIG. 1C shows tables showing genes that are differentially expressed between cases showing favorable prognosis and cases showing poor prognosis and related to an immune system, a cancer pathway, and a kinase in FFPE tissues from DLBCL patients. In the column of "Regulation" in the tables, "up" indicates a gene whose expression increases in patients with poor prognosis, and "down" indicates a gene whose expression decreases in patients with poor prognosis.
FIG. 1D is a diagram showing the number of genes that are differentially expressed between cases showing favorable prognosis and cases showing poor prognosis and related to an immune system, a cancer pathway, and a kinase in FFPE tissue from DLBCL patients.
FIG. 1E is a diagram showing an overall image of gene expression analysis for DLBCL patients when using an nCounter system according to the present invention.
FIG. 1F is a table showing candidate prognostic factor genes extracted by transcriptome analysis.
FIG. 1G is a diagram showing results of gene ontology analysis of candidate prognostic factor genes.
FIG. 2A is a diagram showing a volcano plot of the statistical significance (-log10 p-value from Mann-Whitney U test) and magnitude of fold changes in gene expression level between favorable prognostic cases and poor prognostic cases.
FIG. 2B is a diagram showing survival curves of patients with high or low expression of each gene of MYC, PAICS, FKBP4, PDXP, and GARS.
FIG. 2C is a diagram showing a correlation between PAICS expression and PDXP expression and a correlation between FKBP4 expression and MYC expression, in DLBCL patients.
FIG. 2D is a diagram showing expression of genes of each of MYC, PAICS, FKBP4, PDXP, and GARS, in normal lymph node (LN) samples, DLBCL samples, and DLBCL cell lines.
FIG. 2E is a diagram showing results of gene ontology analysis of candidate favorable prognostic factor genes.
FIG. 3A is a diagram showing expression of IL-21, BCL6, and PD-1 in each fraction of CD4⁺ T cells.
FIG. 3B is a diagram showing expression of IL-21 in CD8⁺ T cells.
FIG. 3C shows a correlation coefficient between expression of IL-21 and expression of a canonical Tfh marker.
FIG. 3D is a view showing results of multiplexed fluorescent immunostaining of each gene of CD20, CD3, and ICOS in DLBCL tissue.
FIG. 3E is a diagram showing a frequency of expression of ICOS genes, in DLBCL patient tissue of relapsed patients or relapse-free patients.
FIG. 3F is a view showing results of multiplexed fluorescent immunostaining of each gene of CD20, CD68, and CD11c in DLBCL tissue.
FIG. 3G is a diagram showing a frequency of expression of CD11c⁺ macrophages in DLBCL patient tissue of relapsed patients or relapse-free patients.
FIG. 4A is a view showing results of multiplexed fluorescent immunostaining of each gene of FGFR1, CD20, CD68, and CD3 in DLBCL tissue.
FIG. 4B is a diagram showing expression of each gene of ICOS, CD11c, and FGFR1 in DLBCL cell lines, DLBCL tissue, and normal lymph nodes.
FIG. 5A is a view showing existence of ICOS, CD11c, and FGFR1, in reactive lymph nodes obtained from patients diagnosed with benign lymphadenopathy.
FIG. 5B is a view showing existence of ICOS, CD11c, and FGFR1 in normal lymph nodes and reactive lymph nodes.
FIG. 5C is a diagram showing a frequency of expression of ICOS, CD11c, and FGFR1 in a GC region of reactive lymph nodes and follicles of normal lymph nodes.
FIG. 5D is a diagram showing survival curves according to presence or absence of expression of ICOS, CD11c, or FGFR1.
FIG. 6A is a diagram showing a method of calculating a DMS score and survival curves based on the calculated DMS score. In FIG. 6A, "pt" represents a "point".
FIG. 6B is a diagram showing a relationship between the DMS score and expression of poor prognosis-associated microenvironment genes.
FIG. 6C is a diagram showing expression of each gene of ICOS, CD11c, and FGFR1, in DLBCL patients.
FIG. 6D is a diagram showing survival curves based on the DMS score.
FIG. 6E is a diagram showing classification of each group of DMS (each group having a DMS score of 0 to 3) by Lymph2Cx and Hans criteria.
FIG. 6F is a diagram showing classification of each group of DMS (each group having a DMS score of 0 to 3) by an IPI risk score.
FIG. 6G is a diagram showing survival curves in classification by Hans criteria and Lymph2Cx.
FIG. 7A is a diagram showing survival curves of each group of DMS, in a group based on Hans criteria.
FIG. 7B is a diagram showing survival curves of each group of DMS, in a group based on Lymph2Cx classification.
FIG. 7C is a diagram showing survival curves of each group of DMS, in a group based on IPI classification.
FIG. 7D is a diagram showing results of multivariate analysis of sex, biopsy site, classification by Hans criteria, classification by Lymph2Cx, an IPI risk score, and a DMS score.
FIG. 7E is a diagram showing results of a permutation test of the DMS score by a random resampling technique.
FIG. 8A is a diagram showing results of unsupervised hierarchical clustering analysis of gene expression profile of immune-related genes.
FIG. 8B is a diagram showing correlations between the DMS score and the expression levels of ICOS, CD11c, and FGFR1, and the number of genes with copy number alteration (CNA).

### BEST MODE FOR CARRYING OUT THE INVENTION

### [Method for Predicting Therapeutic Effectiveness of Chemotherapy for DLBCL Patient]

According to one embodiment, the present invention provides a method for predicting the therapeutic effectiveness of chemotherapy for a DLBCL patient, including: at least one step selected from the group consisting of a step of measuring expression of a marker of a T cell in a sample obtained from the patient, a step of measuring expression of a marker of a macrophage or a dendritic cell in a sample obtained from the patient, and a step of measuring expression of a marker of a stromal cell in a sample obtained from the patient; and a step of predicting prognosis of the treatment, based on the expression of the marker.

In the method for predicting therapeutic effectiveness of chemotherapy for a DLBCL patient of the present embodiment, a sample obtained from the patient is not particularly limited as long as the sample can be used in the method of the present invention. Examples thereof include lymphoma tissue, blood such as peripheral blood, and lymph. The lymphoma tissue obtained from the patient is not particularly limited as long as the lymphoma tissue can be used in the method of the present invention. Examples thereof include formalin-fixed paraffin-embedded (FFPE) tissue.

In the method for predicting therapeutic effectiveness of chemotherapy for a DLBCL patient of the present embodiment, the step of measuring the expression of the marker is not particularly limited as long as the expression of the marker can be measured in the step. Examples of the measurement of the expression of the marker include measurement of an mRNA expression level of the marker and measurement of a protein expression level of the marker, and the measurement of the mRNA expression level of the marker is preferred. Examples of the method for measuring the mRNA expression level of the marker include transcriptome analysis. When the FFPE tissue is used as the lymphoma tissue, the mRNA included in the FFPE tissue is highly fragmented or denatured. Therefore, when the FFPE tissue is used, an nCounter (registered trademark) system, which is one of the transcriptome analysis, is preferred. The nCounter system is a system in which a pair of probes having a barcode sequence that can be identified by a microscope is bound to a target RNA molecule and the number of barcodes is directly counted by the microscope to analyze a large number of genes with high-sensitivity and high-accuracy without any amplification or reverse transcription. Even when the mRNA is fragmented or denatured, it is possible to maintain the sensitivity and the accuracy. As the probe used in the nCounter system, a probe provided by NanoString Technologies, Inc. may be used. Alternatively, the probe may be synthesized by a custom synthesis service provided by NanoString Technologies, Inc. Examples of the method for measuring the protein expression level of the marker include Western blotting and immunohistochemistry. The measurement of the expression of the marker may be performed in a state in which T cells, macrophages or dendritic cells, or stromal cells are included in the sample (for example, lymphoma tissue), and may be performed after these cells are isolated from the sample (for example, peripheral blood).

In the method for predicting therapeutic effectiveness of chemotherapy for a DLBCL patient of the present embodiment, when the step of measuring the expression of the marker is a step of measuring the mRNA expression level of the marker, examples of the step of predicting the prognosis of the treatment based on the expression of the marker include a step of indicating that the prognosis of the treatment is favorable, when the mRNA expression level of the marker is equal to or more than a cutoff value determined by receiver operating characteristic analysis (hereinafter, referred to as ROC analysis). Alternatively, when the step of measuring the expression of the marker is a step of measuring the mRNA expression level of the marker, examples of the step of predicting the prognosis of the treatment based on the expression of the marker include a step of indicating that the prognosis of the treatment is poor, when the mRNA expression level of the marker is less than the cutoff value determined by the ROC analysis. The cutoff value can be determined by obtaining an area under a ROC curve by the ROC analysis and maximizing Youden index therein. Examples of prognosis include probability of progression-free survival (PFS), progression-free survival time, probability of overall survival (OS), survival time, probability of event-free survival (EFS), and event-free survival time.

For example, when calculating is performed by setting a score to 1 when the expression level of each mRNA of the marker is equal to or more than the cutoff value determined by the ROC analysis and setting the score to 0 when the expression level is less than the cutoff value, a higher sum of the scores can indicate more favorable prognosis. In the present specification, a score calculated by setting the score to 1 when the expression level of each mRNA of the marker is equal to or more than the cutoff value determined by the ROC analysis and setting the score to 0 when the expression level is less than the cutoff value is referred to as a DLBCL microenvironment signature (DMS) score.

In the method for predicting therapeutic effectiveness of chemotherapy for a DLBCL patient of the present embodiment, examples of the chemotherapy include anti-CD20 antibody monotherapy and chemotherapy in combination with anti-CD20 antibody. The chemotherapy in combination with anti-CD20 antibody refers to chemotherapy using an anti-CD20 antibody and another chemotherapeutic agent in combination, and examples thereof include R-CHOP therapy, G-CHOP therapy, CHASER therapy, R-THP-COP therapy, and DA-EPOCH-R therapy.

Examples of the anti-CD20 antibody used in the chemotherapy in chemotherapy in combination with anti-CD20 antibody include rituximab (product name: Rituxan (registered trademark)), ofatumumab (product name: Arzerra (registered trademark)), obinutuzumab (product name: Gazyba (registered trademark)), Ibritumomab tiuxetan (product name: Zevalin (registered trademark)), ocrelizumab, and veltuzumab.

The other chemotherapeutic agents used in combination with the anti-CD20 antibody include cyclophosphamide (product name: Endoxan (registered trademark)), cytarabine (product name: kiloside (registered trademark)), etoposide (product name: Lastet (registered trademark)), dexamethasone (product name: Dexart (registered trademark)), doxorubicin, vincristine (product name: Oncovin (registered trademark)), prednisolone (product name: Predonine (registered trademark)), pirarubicin (product name: Therarubicin (registered trademark)), and bendamustine (Treakisym (registered trademark)).

The CHOP therapy is a treatment method using cyclophosphamide, doxorubicin, vincristine, and prednisolone, and the R-CHOP therapy is a treatment method in which the rituximab is further added to the CHOP therapy. The G-CHOP therapy is a treatment method in which the obinutuzumab is further added to the CHOP therapy. The CHASER therapy is a treatment method using cyclophosphamide, cytarabine, etoposide, dexamethasone, and rituximab. The R-THP-COP therapy is a treatment method using rituximab, pirarubicin, cyclophosphamide, vincristine, and prednisolone. The DA-EPOCH-R therapy is a treatment method using etoposide, vincristine, cyclophosphamide, doxorubicin, prednisone, and rituximab.

In the method for predicting therapeutic effectiveness of chemotherapy for a DLBCL patient of the present embodiment, examples of the marker of the T cell include CCR4, CD2, CD3D, CD3E, CD6, CD7, CD96, FAS, IL2, IL2RB, IL2RG, IL7R, ITK, ITPKB, TNFRSF9, TRAF1, CD40LG, CTLA4, CXCL13, ICOS, IL21, PDCD1, SH2D1A, SLAMF1, and TNFRSF4. These markers may be used alone, or a plurality of these may be used in combination.

In addition, the T cell is not particularly limited as long as the T cells can be used in the method of the present invention, and follicular helper T cell (hereinafter, referred to as Tfh cell) is preferable. Examples of the marker of the Tfh cell include CD40LG, CTLA4, CXCL13, ICOS, IL21, PDCD1, SH2D1A, SLAMF1, and TNFRSF4. These markers may be used alone, or a plurality of these may be used in combination.

In the method for predicting therapeutic effectiveness of chemotherapy for a DLBCL patient of the present embodiment, examples of the marker of the macrophage or the dendritic cell include CD80, HLA-DRB1, CD11c (ITGAX), and NOD2. These markers may be used alone, or a plurality of these may be used in combination.

In the method for predicting therapeutic effectiveness of chemotherapy for a DLBCL patient of the present embodiment, examples of the marker of the stromal cell include BMP2K, COL8A2, FGFR1, and OBSCN. These markers may be used alone, or a plurality of these may be used in combination.

In the method for predicting therapeutic effectiveness of chemotherapy for a DLBCL patient of the present embodiment, any one of the step of measuring expression of the marker of the T cell in the sample obtained from the patient, the step of measuring expression of the marker of the macrophage or the dendritic cell in the sample obtained from the patient, and the step of measuring expression of the marker of the stromal cell in the sample obtained from the patient may be performed, and two or more steps thereof may be performed in combination. It is preferable that two or more steps thereof be combined, and it is more preferable that all three steps be combined.

In the method for predicting therapeutic effectiveness of chemotherapy for a DLBCL patient of the present embodiment, examples of a method in which the three steps, that is, the step of measuring expression of the marker of the T cell in the sample obtained from the patient, the step of measuring expression of the marker of the macrophage or the dendritic cell in the sample obtained from the patient, and the step of measuring expression of the marker of the stromal cell in the sample obtained from the patient are combined, include a method in which steps of measuring expression of ICOS as the marker of the T cell, CD11c as the marker of the dendritic cell, and FGFR1 as the marker of the stromal cell are combined.

Specific examples of the method for predicting therapeutic effectiveness of chemotherapy for a DLBCL patient of the present embodiment, in which the steps of measuring mRNA expression levels of the ICOS, CD11c, and FGFR1 are combined, can include a method in which the DMS score is calculated by measuring the expression level of each mRNA of the ICOS, the CD11c, and FGFR1, and setting the score of each marker to 1 when the expression levels of the ICOS, CD11c, and FGFR1 are equal to or more than cutoff values determined by the ROC analysis, specifically, are equal to or more than 543.65, 2428.88, and 886.37, respectively, and when setting the score of each marker to 0 when the expression levels are less than the values, a higher sum of the scores can indicate more favorable prognosis. In this method, as the DMS score increases to 0, 1, 2, and 3, the prognosis (for example, PFS or OS) of the treatment is indicated to be better.

### [Kit for Predicting Therapeutic Effectiveness of Chemotherapy for DLBCL Patient]

According to one embodiment, the present invention provides a kit for predicting therapeutic effectiveness of chemotherapy for a DLBCL patient, including: at least one reagent selected from the group consisting of a reagent for measuring expression of a marker of a T cell, a reagent for measuring expression of a marker of a macrophage or a dendritic cell, and a reagent for measuring expression of a marker of a stromal cell.

In the kit for predicting therapeutic effectiveness of chemotherapy for a DLBCL patient of the present embodiment, examples of the chemotherapy include chemotherapy in combination with anti-CD20 antibody. Examples of the chemotherapy in combination with anti-CD20 antibody include R-CHOP therapy, G-CHOP therapy, CHASER therapy, R-THP-COP therapy, and DA-EPOCH-R therapy. As the anti-CD20 antibody and other chemotherapeutic agents used in combination with the anti-CD20 antibody, those exemplified in the method for predicting the therapeutic effectiveness of chemotherapy for a DLBCL patient are used.

In the kit for predicting therapeutic effectiveness of chemotherapy for a DLBCL patient of the present embodiment, the reagent for measuring the expression of the marker of the T cell is not particularly limited as long as the reagent is used for measuring the expression of the marker of the T cell, and examples thereof include fluorescent-labeled probe in which a probe designed to bind to the marker of the T cell is labeled with fluorescence or the like. Examples of the probe include a nucleic acid probe such as DNA and RNA, and a binding molecule such as antibody and antibody fragment.

The marker of the T cell used in the kit include CCR4, CD2, CD3D, CD3E, CD6, CD7, CD96, FAS, IL2, IL2RB, IL2RG, IL7R, ITK, ITPKB, TNFRSF9, TRAF1, CD40LG, CTLA4, CXCL13, ICOS, IL21, PDCD1, SH2D1A, SLAMF1, and TNFRSF4, and these markers may be used alone, or a plurality of these may be used in combination.

In addition, the T cell is not particularly limited as long as the T cell can be used in the kit of the present invention, and Tfh cell is preferable. Examples of the marker of the Tfh cell include CD40LG, CTLA4, CXCL13, ICOS, IL21, PDCD1, SH2D1A, SLAMF1, and TNFRSF4. These markers may be used alone, or a plurality of these may be used in combination.

In the kit for predicting therapeutic effectiveness of chemotherapy for a DLBCL patient of the present embodiment, the reagent for measuring the expression of the marker of the macrophage or the dendritic cell is not particularly limited as long as the macrophage or the dendritic cell is used for measuring the marker of the macrophage or the dendritic cell, and examples thereof include a fluorescent-labeled probe in which a probe designed to bind to the marker of the macrophage or the dendritic cell is labeled with fluorescence or the like. Examples of the probe include a nucleic acid probe such as DNA and RNA, and a binding molecule such as antibody and antibody fragment.

In the kit for predicting therapeutic effectiveness of chemotherapy for a DLBCL patient of the present embodiment, examples of the marker of the macrophage or the dendritic cell include CD80, HLA-DRB1, CD11c (ITGAX), and NOD2. These markers may be used alone, or a plurality of these may be used in combination.

In the kit for predicting therapeutic effectiveness of chemotherapy for a DLBCL patient of the present embodiment, the reagent for measuring the expression of the marker of the stromal cell is not particularly limited as long as the reagent is used for measuring the expression of the marker of the stromal cell, and examples thereof include fluorescent-labeled probe in which a probe designed to bind to the marker of the stromal cell is labeled with fluorescence or the like. Examples of the probe include a nucleic acid probe such as DNA and RNA, and a binding molecule such as antibody and antibody fragment.

In the kit for predicting therapeutic effectiveness of chemotherapy for a DLBCL patient of the present embodiment, examples of the marker of the stromal cell include BMP2K, COL8A2, FGFR1, and OBSCN. These markers may be used alone, or a plurality of these may be used in combination.

The kit for predicting therapeutic effectiveness of chemotherapy for a DLBCL patient of the present embodiment may further include another reagent in addition to the reagent for measuring the expression of the marker of the T cell, the reagent for measuring the expression of the marker of the macrophage or the dendritic cell, and the reagent for measuring the expression of the marker of the stromal cell. Examples of the other reagents include reagents used in the nCounter system and reagents used in immunohistochemistry and Western blotting (for example, buffer solution, washing solution, and detection reagent).

In addition, the kit for predicting therapeutic effectiveness of chemotherapy for a DLBCL patient of the present embodiment may further include instructions for calculating the DMS scores. The cutoff value or the like of each marker may be described in the instructions.

Further, the expression level of each marker measured using the kit for predicting therapeutic effectiveness of chemotherapy for a DLBCL patient of the present embodiment is input to a device such as a computer via a network such as Internet or intranet. The device calculates the DMS score using the cutoff value of each marker. The therapeutic effectiveness of the chemotherapy is predicted by the numerical values of the DMS scores. The predicted results can be output to a device such as another computer or tablet via the network.

The kit for predicting therapeutic effectiveness of chemotherapy for a DLBCL patient of the present embodiment can be used for the method for predicting therapeutic effectiveness of chemotherapy for a DLBCL patient of the present invention.

In addition, when in the method for predicting therapeutic effectiveness of chemotherapy for a DLBCL patient, the prognosis is predicted to be favorable, the present invention provides a treatment method for the DLBCL patient, including administering the chemotherapy to the DLBCL patient.

When in the method for predicting therapeutic effectiveness of chemotherapy for a DLBCL patient of the present embodiment, the prognosis is predicted to be favorable, the prognosis of the DLBCL patient can be made favorable by performing the chemotherapy and treating the DLBCL patient.

The present invention provides a marker of a T cell, a marker of macrophage or a dendritic cell, and a marker of a stromal cell in a sample, which are used in the method for predicting therapeutic effectiveness of chemotherapy for a DLBCL patient, including at least one step selected from the group consisting of a step of measuring expression of a marker of a T cell in a sample obtained from the patient, a step of measuring expression of a marker of a macrophage or a dendritic cell in a sample obtained from the patient, and a step of measuring expression of a marker of a stromal cell in a sample obtained from the patient; and a step of predicting prognosis of the treatment, based on the expression of the marker.

In addition, the present invention provides use of a marker of a T cell, a marker of macrophage or a dendritic cell, and a marker of a stromal cell in a sample, for manufacturing the kit for predicting therapeutic effectiveness of chemotherapy for a DLBCL patient. Furthermore, the present invention provides use of at least one reagent selected from the group consisting of a reagent for measuring expression of a marker of a T cell, a reagent for measuring expression of a marker of a macrophage or a dendritic cell, and a reagent for measuring expression of a marker of a stromal cell, for manufacturing a kit for predicting therapeutic effectiveness of chemotherapy for a DLBCL patient.

### EXAMPLES

Hereinafter, the present invention will be described more specifically with reference to Examples or the like, but the present invention is not limited to the following Examples.

### (Example 1) Gene Expression of Candidate Prognostic Factor Gene

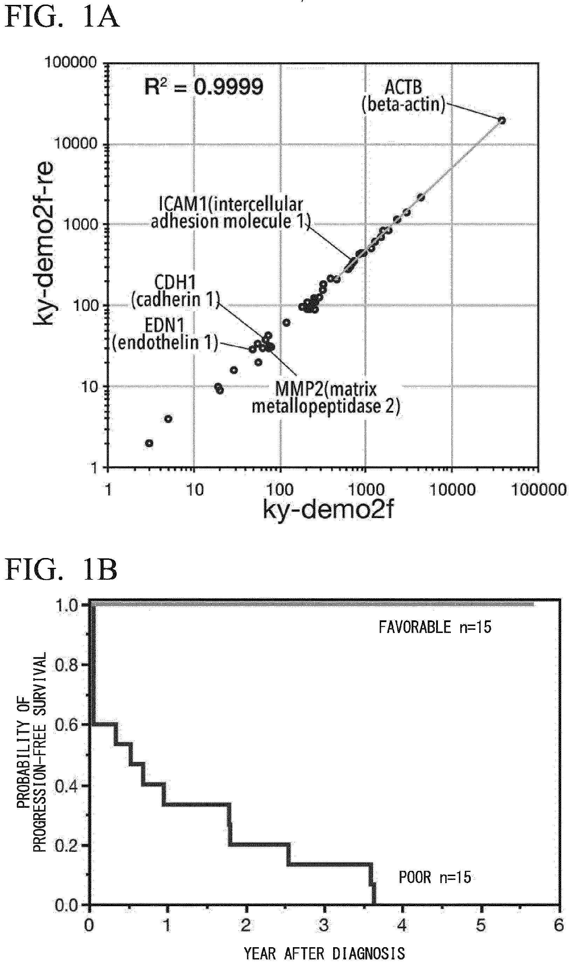

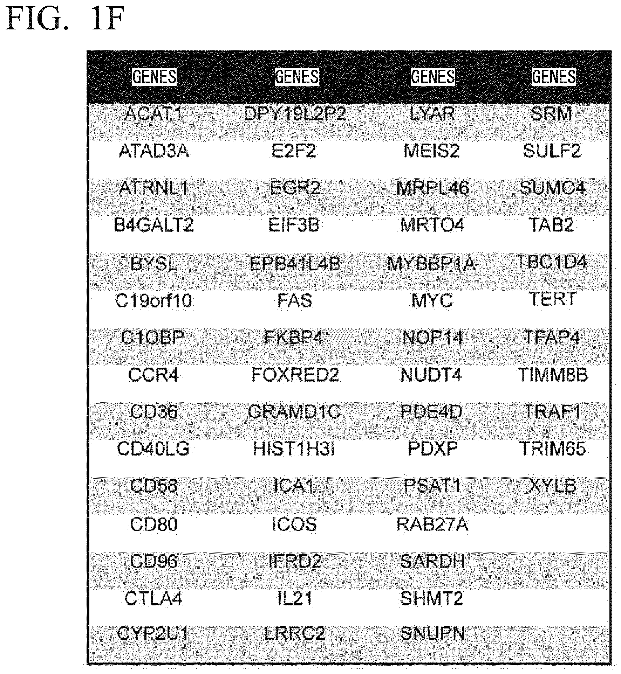

Expression levels of 48 representative genes (shown in Table 1 below) of FFPE samples from DLBCL patients were analyzed using the nCounter system. The results thereof are shown in FIG. 1A. As shown in FIG. 1A, it was confirmed that even lowly-expressed transcripts presumably derived from rare microenvironmental components were quantitatively measured with extremely high reproducibility.

**[Table 1]**

| Gene | Gene | Gene |
|---|---|---|
| ABCC4 | HBEGF | PSMC4 |
| ACTB | HDAC1 | PTGS2 |
| ADM | HIF1A | RNF10 |
| AMD1 | HIST1H1D | SEC24D |
| APC | ICAM1 | SFRS10 |
| ASPA | IGF2R | SHCBP1 |
| BTBD15 | IL1B | STAT6 |
| C11orf58 | ITM2B | TDG |
| C13orf23 | KLF10 | TFRC |
| CCNA2 | LDHA | THBS1 |
| CDH1 | LGI1 | TNFAIP3 |
| CHGB | MMP2 | TP53 |
| CYR61 | NDUFV3 | TRAF4 |
| DNAJB9 | NTS | TYMS |
| EDN1 | POLR1B | UBE2B |
| EXOC2 | PPFIA2 | ZNF434 |

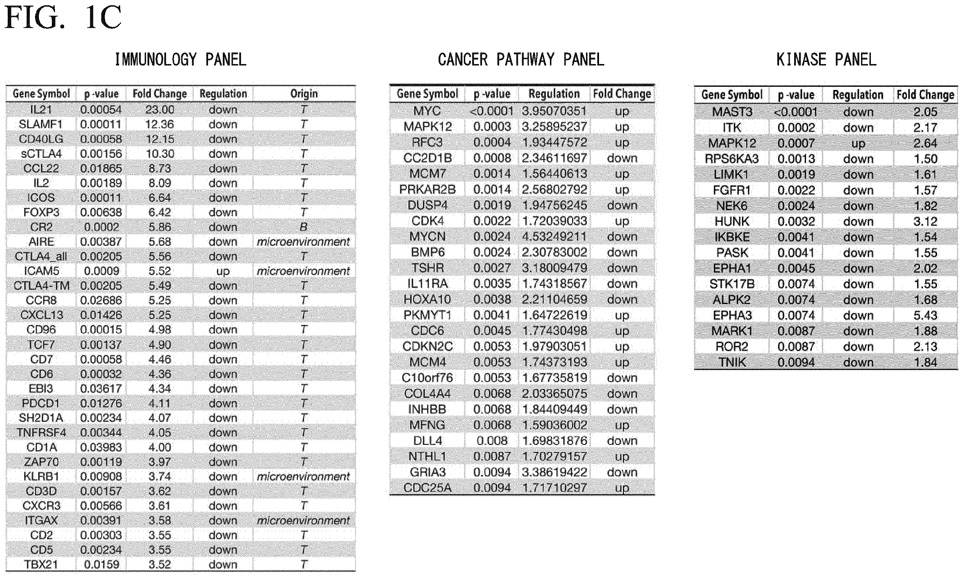

Next, R-CHOP therapy was performed, and expression levels of 1900 genes related to an immune system, a cancer pathway, and a kinase were examined using FFPE tissue from 30 DLBCL cases including 15 DLBCL patients of each favorable and poor courses (FIG. 1B), using the nCounter system. Results thereof are shown in FIG. 1C. As shown in FIG. 1C, genes that are differentially expressed with statistically significance between cases exhibiting favorable prognosis and those with poor prognosis were identified.

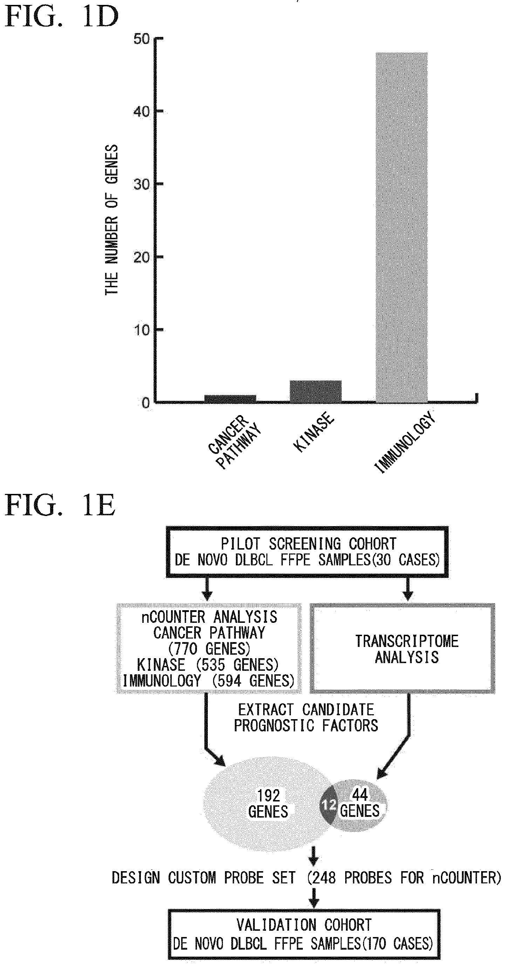

The genes that are differentially expressed with statistically significance between cases exhibiting favorable prognosis and those with poor prognosis were enriched in immunology panels (FIG. 1D). Most of them were not tumor B cell origin, but microenvironment-related, including genes expressed in T cells, genes expressed in NK cells (KLRB1), and genes expressed in other microenvironment cells (ITGAX, AIRE). Other microenvironment-related prognostic genes were FGFR1 (fibroblast growth factor receptor 1) and COL4A4 (collagen type IV alpha 4). MYC, well-known oncogenes with a significant role in a cell cycle, apoptosis, and cellular transformation, were extracted as a top-ranked prognostic factor from the cancer pathway panel (FIG. 1C).

Since the number of analyzed genes by the nCounter system is a maximum of 800 genes per assay, transcriptome analysis by RNA-sequencing was performed to cover up genes not analyzed in screening with the nCounter system (FIG. 1E). As a result, 56 genes, which were differentially expressed in the favorable prognosis group and poor prognosis group, were extracted as candidate prognostic factors (FIG. IF). Among the 56 genes, 12 genes were genes extracted in common with the screening with the nCounter system.

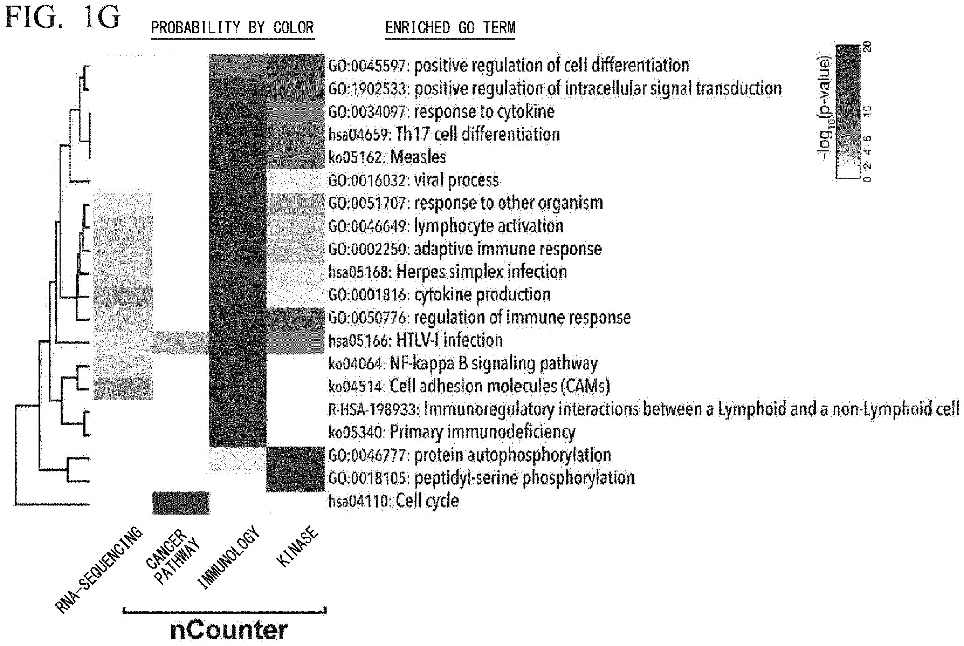

Based on the results of two screenings in the pilot cohort, 248 candidate prognostic factor genes were selected, and probe sets for validation cohort with the nCounter system were designed by custom synthesis (NanoString Technologies, Inc.). As a result of gene ontology (hereinafter, also referred to as GO) analysis, it was found that these candidate genes were mostly enriched in the immune system (FIG. 1G). This suggests that an immune status was highly associated with clinical outcome of DLBCL patients.

### (Example 2) Identification of Poor Prognostic Factor

The gene expression of the candidate prognostic factor genes identified using the nCounter system in Example 1 was analyzed, in 170 DLBCL samples treated with R-CHOP or R-CHOP-like chemotherapy between 2006 and 2013 (Age, median 71 y.o. [range, 23-89 y.o.]; IPI (international prognostic index): low risk and low-intermediate risk 51.5%, high-intermediate risk and high risk 48.3%; Observation period, median 3.3 years).

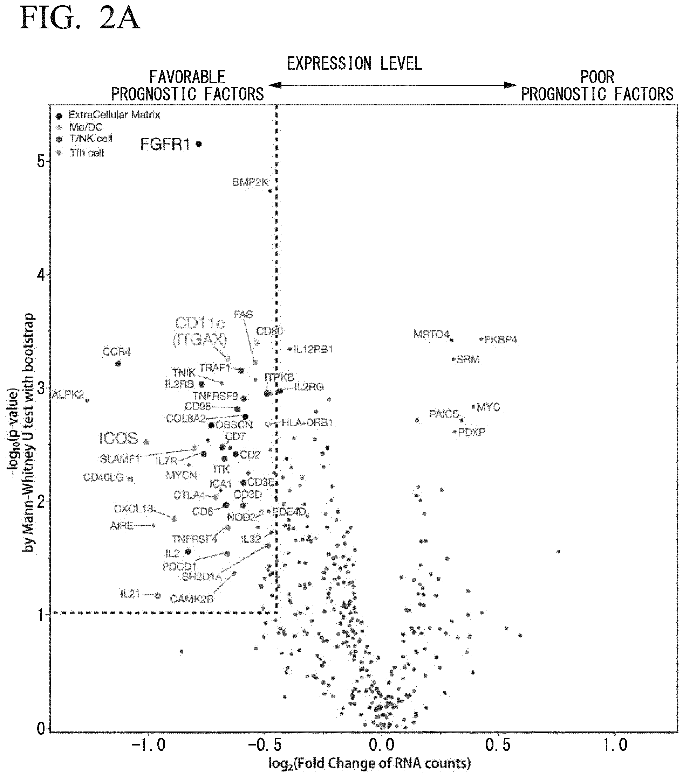

As a result, several prognostic factors were identified in the volcano plot of the statistical significance (-log10p-value from Mann-Whitney U test) and a magnitude of fold changes in gene expression level between favorable prognostic cases and poor prognostic cases (FIG. 2A).

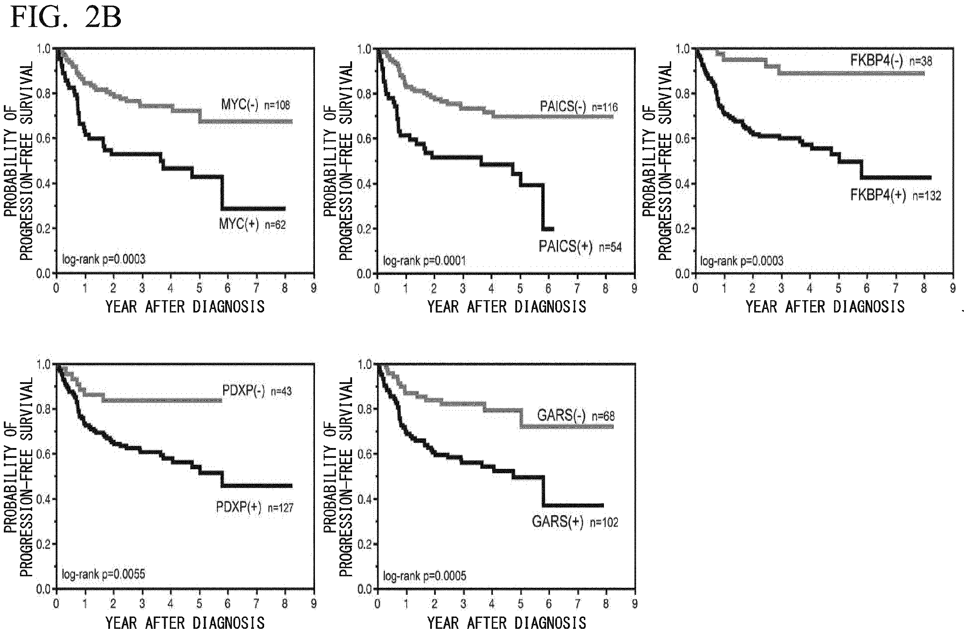

One of the most significant poor prognostic factors was MYC. The status of the MYC, not only gene rearrangement status but also protein expression have been reported to predict clinical outcome. Consistent with these reports, a high level of MYC mRNA expression strongly predicted poor survival (FIG. 2B).

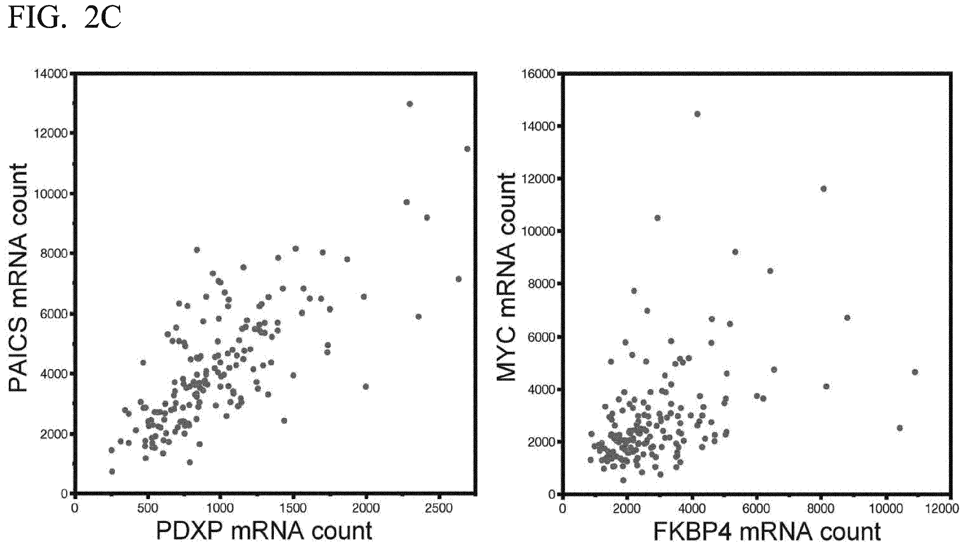

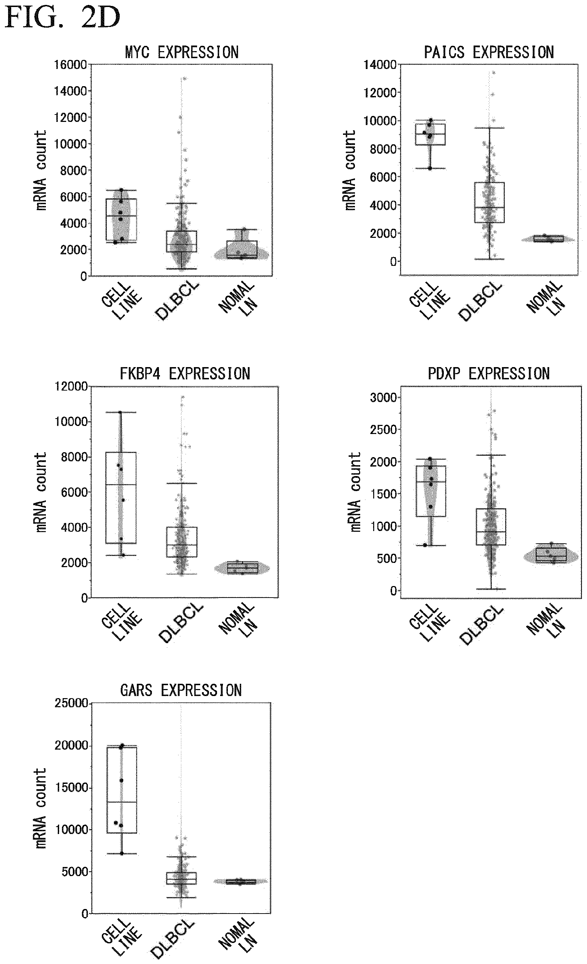

Other poor prognostic factors were phosphoribosylaminoimidazole carboxylase and phosphoribosylaminoimidazolesuccinocarboxamide synthase (PAICS), which plays an important role in de novo purine biosynthesis, glycyl-tRNA synthetase (GARS) and pyridoxal phosphatase (PDXP) related to Vitamin B6 metabolism (FIG. 2B). Upregulation of these genes was co-occurred (FIG. 2C). The upregulation of these genes indicates high activity of lymphoma cells. In fact, the expression of these genes was most upregulated in DLBCL cell lines, followed by DLBCL samples, and normal lymph node samples (FIG. 2D), and therefore may be potential therapeutic targets.

### (Example 3) Identification of Favorable Prognostic Factor

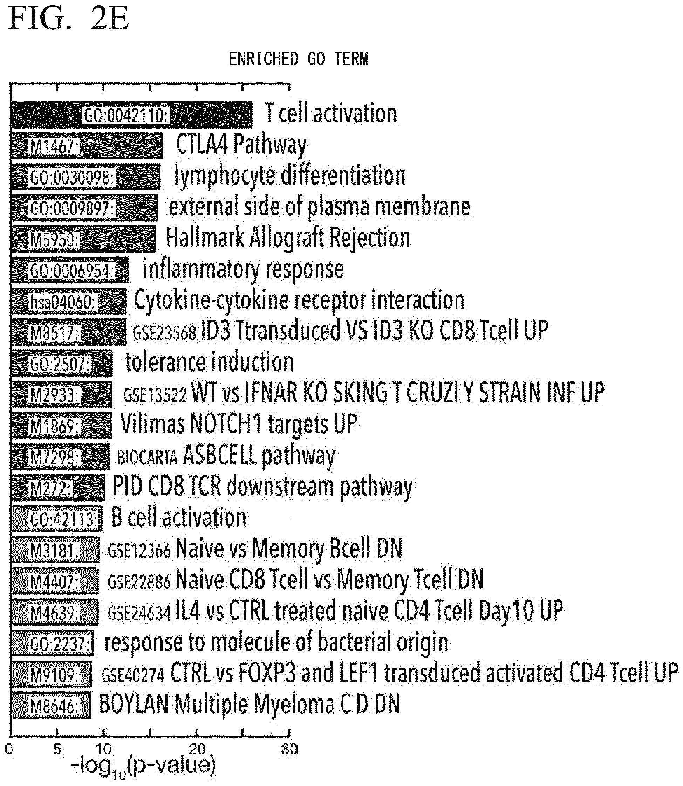

As in Example 2, favorable prognostic factors, which were upregulated in patients with favorable clinical course, were identified in terms of both p-value and fold change (FIG. 2A). These favorable prognostic factors were enriched in immune-related GO terms, especially in immune interaction terms (FIG. 2E). Most favorable prognostic factors were T cell- or NK-cell-related genes, macrophage- or dendritic cell (hereinafter, also referred to as macrophage/DC)-related genes, extracellular matrix (ECM)-related genes, which are presumably expressed by tumor microenvironment components.

Among these, attention was focused on ICOS, as a key marker for Tfh cells; CD11c, as a key marker for macrophage/DC; and FGFR1, as a key marker for stromal cells, and to confirm whether these genes were co-expressed by identical cells, ultrasensitive single-cell GEP of infiltrated T cells was performed by utilizing C1 system (produced by Fluidigm) and Biomark (registered trademark) system (produced by Fluidigm).

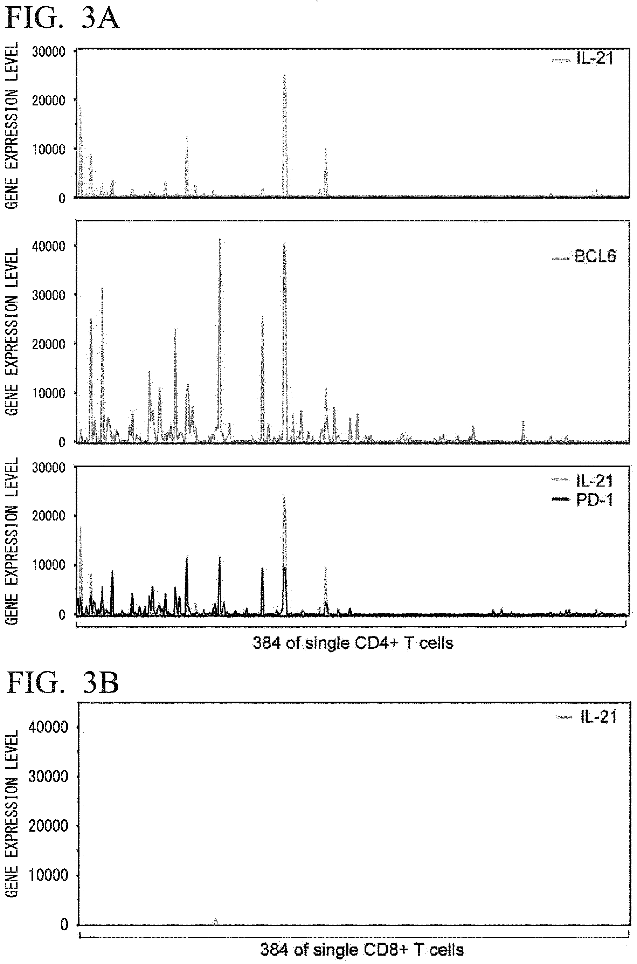

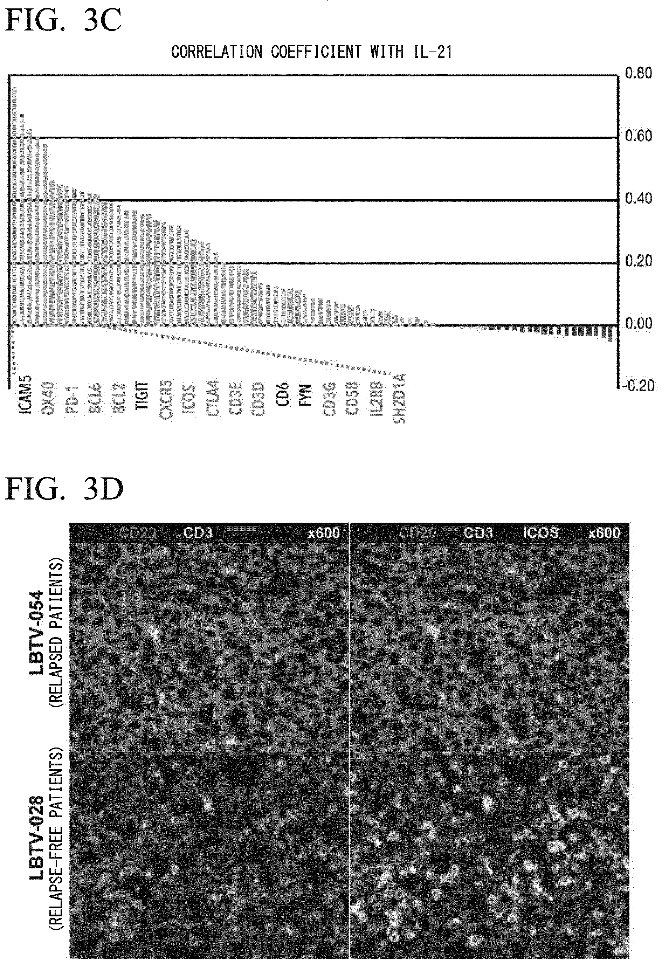

As a result, IL-21, which is specific marker for the Tfh cells and is specified as one of the prognostic factors in Examples 1 and 2, was expressed exclusively in a rare fraction of CD4⁺ T cells (FIG. 3A), in none of CD8⁺ T cells (FIG. 3B), and co-expressed with canonical Tfh markers such as OX40, PD-1, BCL-6, PD-1, CXCR5, ICOS and SLAMF1 (FIG. 3C). This suggests that the Tfh cells are one of the key components predicting favorable prognosis in DLBCL microenvironment.

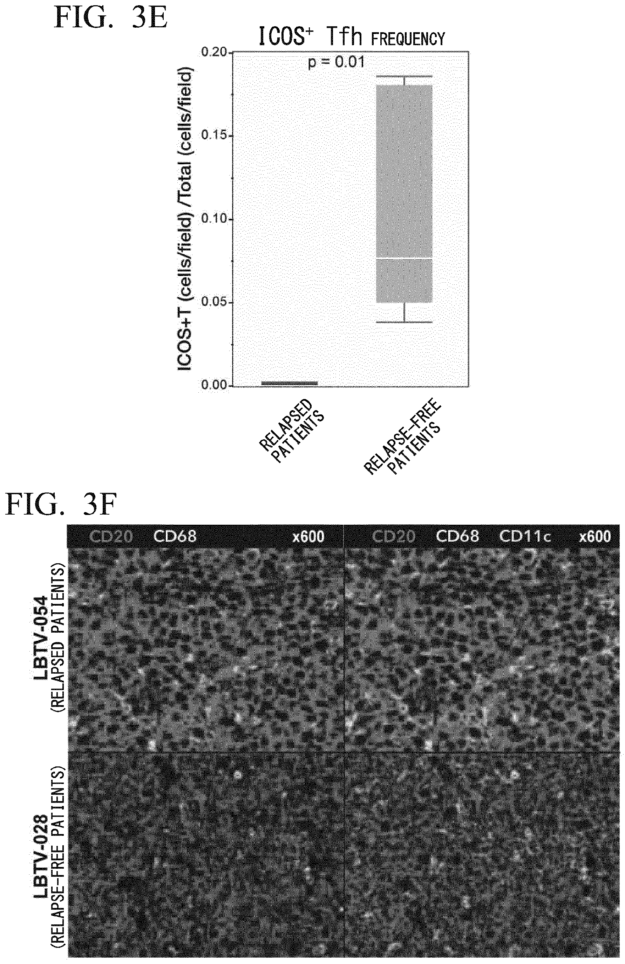

Moreover, a visualization of DLBCL tissue with multiplexed fluorescent immunostaining using quantitative pathology workstation (Mantra (Registered trademark), produced by Perkin-Elmer) was performed and confirmed that ICOS⁺ Tfh cells were infiltrated more significantly in relapse-free patient samples (FIGS. 3D and 3E).

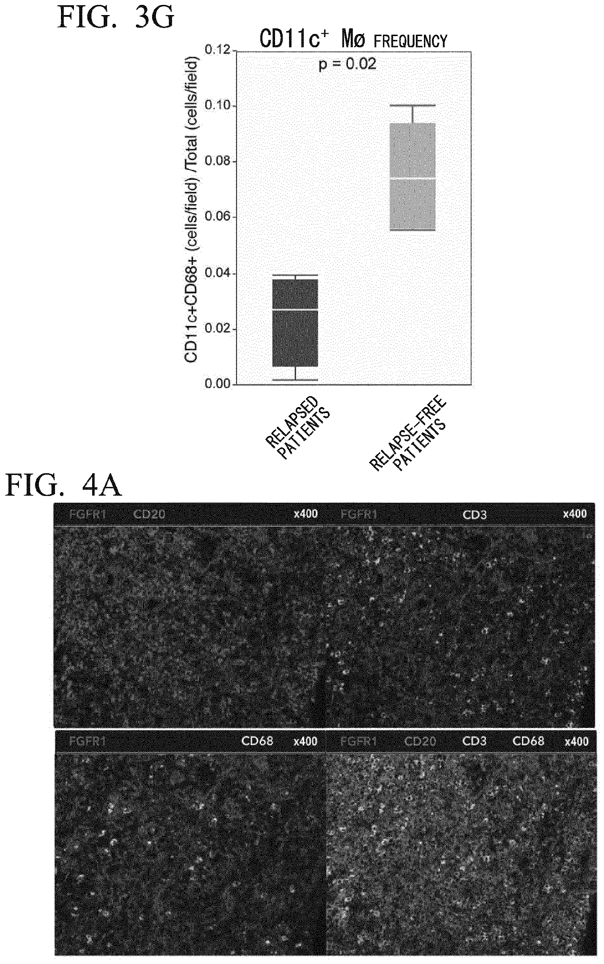

In multiplexed fluorescent immunostaining images of DLBCL tissue, CD11c-positive cells perfectly matched with CD68⁺ cells, but not with CD3⁺ or CD20⁺ cells (FIG. 3F). This suggests that this prognostic factor derived from CD11c⁺ CD68⁺ macrophage/DC was a major factor in GC microenvironment serving as scavengers of apoptotic B cells. Quantitative analysis for these cell components visually confirmed that the enrichment of CD11c⁺ CD68⁺ macrophage/DCs was associated with long-term relapse-free survival (FIG. 3G).

The positive cells for the other microenvironment-related favorable prognostic genes, FGFR1 (fibroblast growth factor 1), did not merge with any lineage-specific markers such as CD20, CD68, CD11c and CD3, residing in intercellular space (FIG. 4A). This confirmed that the FGFR1 is ECM-related gene.

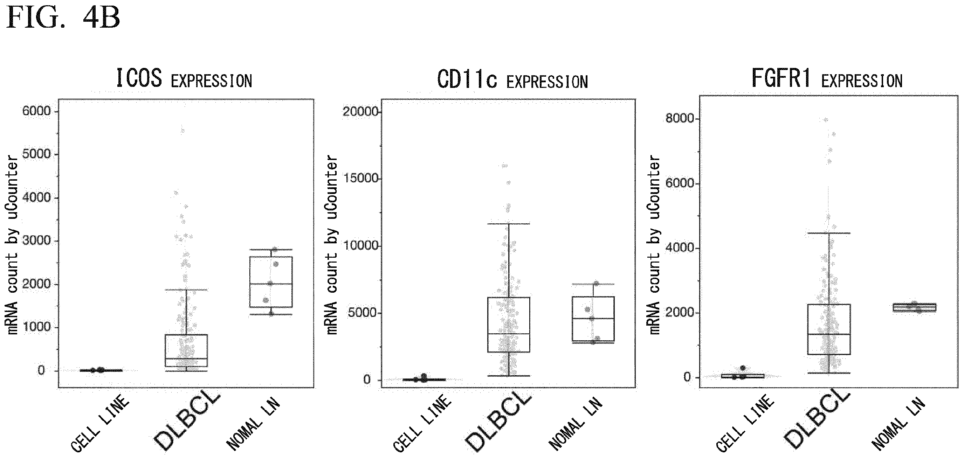

No expression of these prognostic factors was observed in DLBCL cell lines (FIG. 4B). From this fact, it was considered that these prognostic factors were derived from microenvironment in DLBCL tissues. Furthermore, lower expression in the DLBCL samples compared to normal lymph nodes indicated that the loss of specific microenvironment signatures such as Tfh cells, CD11c⁺ CD68⁺ macrophages/DCs, and FGFR1⁺ stromal cells was associated with the generation of DBLCL.

### (Example 4) Relationship between Tissue-of-Origin Based on Microenvironment and Clinical Outcome of DLBCL Patients

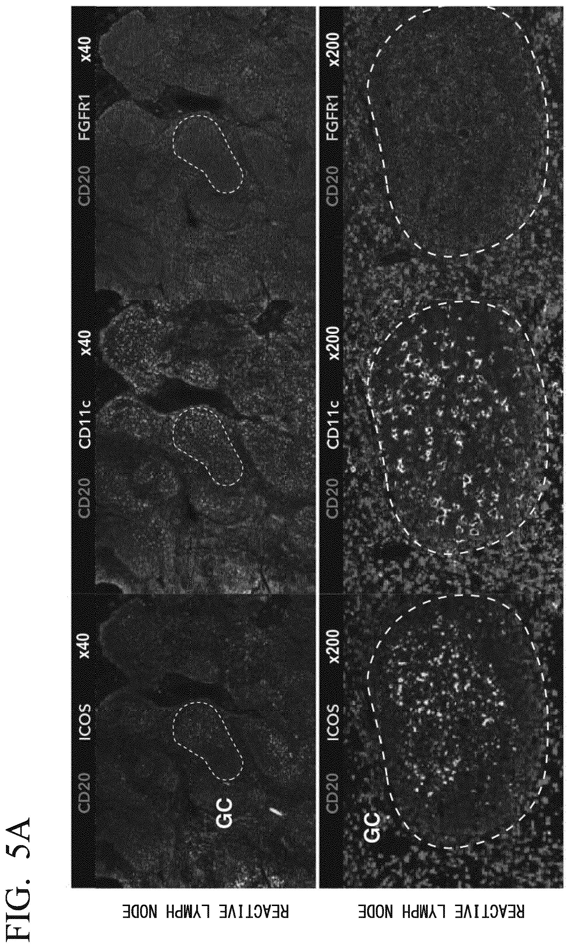

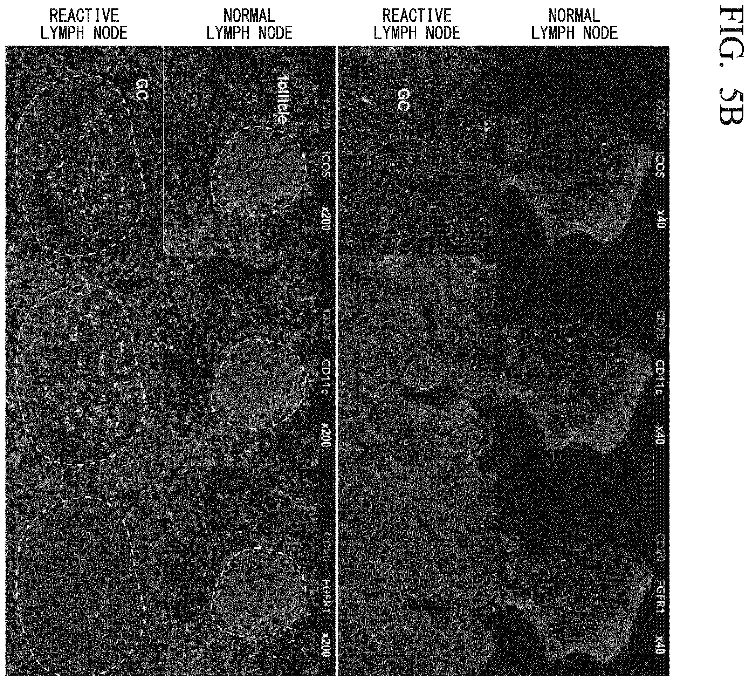

Using quantitative pathology workstation (Mantra (Registered trademark), produced by Perkin-Elmer), the existence of the microenvironment-derived prognostic factors in reactive lymph nodes obtained from patients diagnosed with benign lymphadenopathy was analyzed (FIG. 5A). As a result, ICOS⁺ Tfh cells, CD11c⁺CD68⁺ macrophages/DCs, and FGFR1⁺ stromal cells existed quite evidently in a GC region. Little or no expression of these genes was found in B-cell follicles of normal lymph nodes without GC (FIG. 5B).

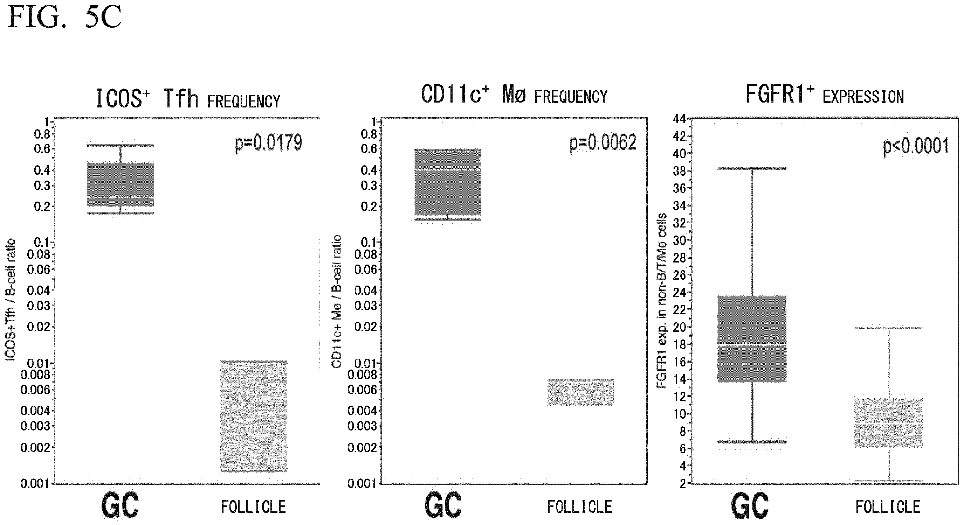

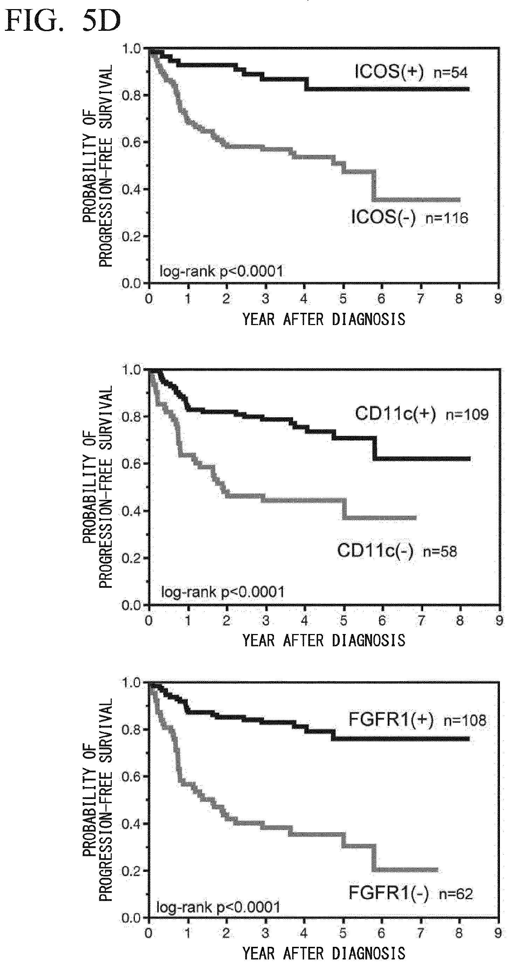

Digital quantification analysis confirmed that these prognostic microenvironment cells were enriched in the GC region (FIG. 5C). These results suggested that the enrichment of these microenvironment cells reflects the GC signature and defines the origin of DLBCL tissue, the so-called "tissue-of-origin", but not of lymphoma cells. Furthermore, as shown in FIG. 5D, these GC microenvironment cells were themselves strong predictors for prognosis of DLBCL patients.

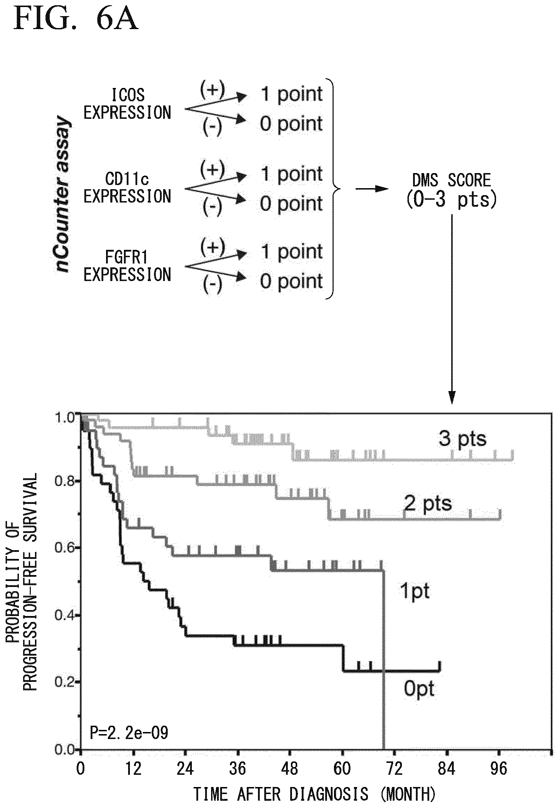

Based on this "tissue-of-origin" concept, DMS score, which is a novel scoring system representing lymphoma microenvironment, was established. This system is defined by the presence of three components of GC microenvironment. Examples of the DMS score include a score calculated by the expression status of ICOS, CD11c, and FGFR1 from nCounter, as shown in FIG. 6A. The DMS score stratified DLBCL patients, clearly reflecting prognosis of the patients.

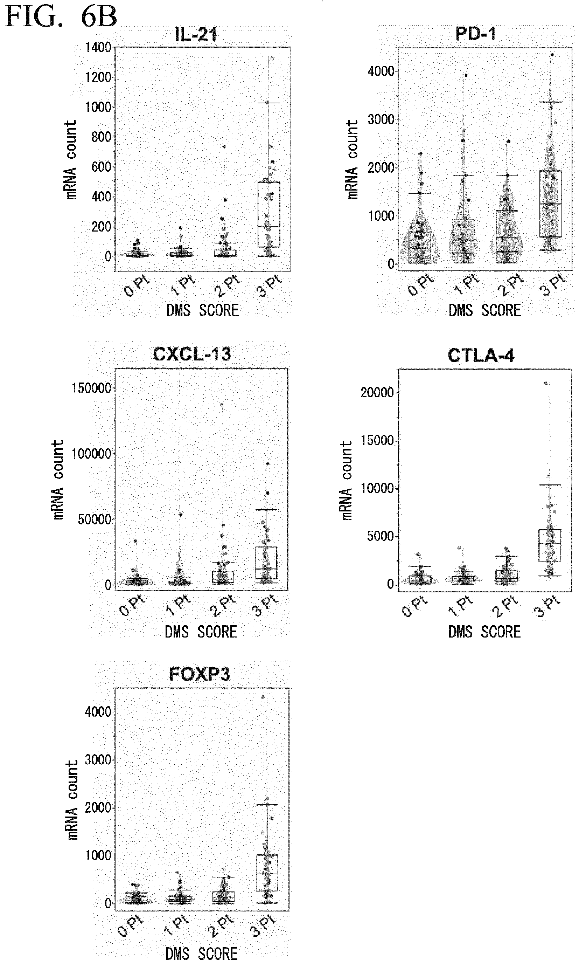

As described above, the enrichment of these Tfh-related markers in a group with a high DMS score was confirmed, while IL-21, PD-1, CXCL-13, CTLA-4, and FOXP3, which are poor prognosis-associated microenvironment genes, were not upregulated or were downregulated (FIG. 6B).

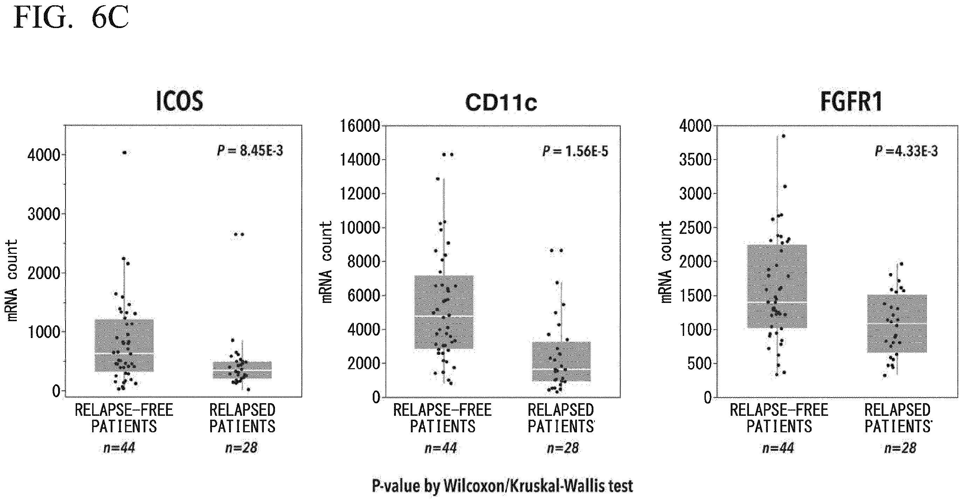

Furthermore, 72 cases of DLBCL patients treated with the R-CHOP therapy were added, and the relationship between the clinical outcome and the expression level of each gene of ICOS, CD11c, and FGFR1 was examined in the same manner as described above. As a result, the expression of genes of ICOS, CD11c, and FGFR1 was increased in the relapse-free patients, whereas the expression of none of these genes increased in the relapsed patients (FIG. 6C). These results also indicate that ICOS, CD11c, and FGFR1 are strong predictors of the prognosis of DLBCL patients.

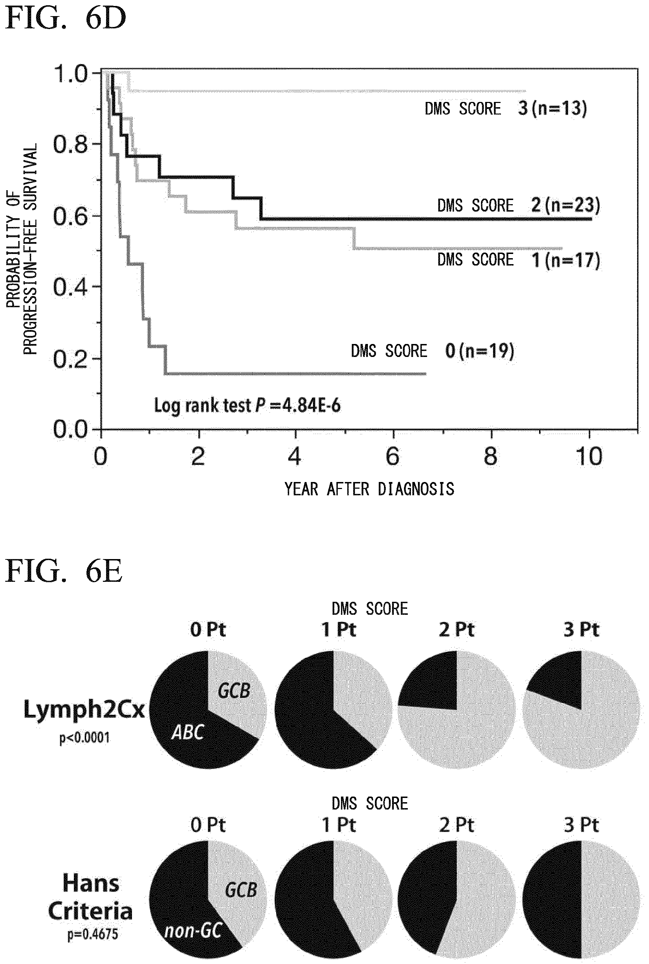

In addition, in these 72 cases, DMS scores were calculated and a survival curve was drawn by the Kaplan-Meier method (FIG. 6D). As is clear from FIG. 6D, the higher the DMS score, the higher the progression-free survival. Therefore, it was confirmed that the DMS score stratifies DLBCL patients to clearly reflect the prognosis of the patients.

Each group of DMS (each group having a DMS score of 0 to 3) contains a GCB type and a non-GC type, which were assigned by canonical COO classification by Hans criteria, with the constant proportion and no consistent correlation was observed (FIG. 6E). On the other hand, GCB cases assigned by a refined COO classification model, Lymph2Cx score, were enriched in groups having a high DMS score with statistical significance. This suggests that the DMS score and the Lymph2Cx both reflected GC-like signature from the viewpoint of microenvironment and lymphoma cell signature, respectively.

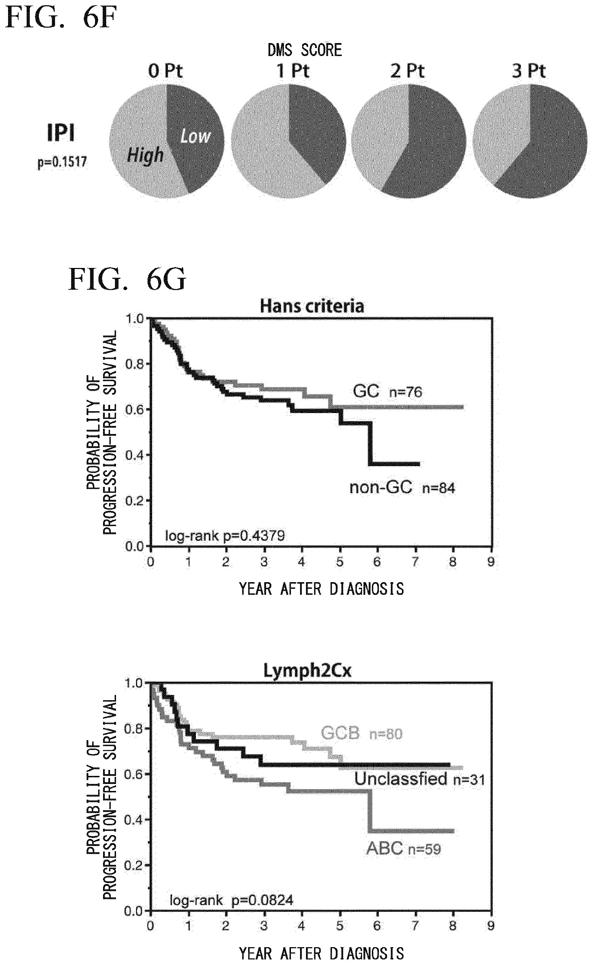

Even though a correlation between subgroups by IPI risk and those by DMS score was seen (FIG. 6F), the DMS score could predict the prognosis of patients more accurately than canonical COO-based stratification models (FIGS. 6A, 6D, and 6G). Then, it was examined whether the DMS score could add to the prognostic value of canonical clinical prognostic indicators.

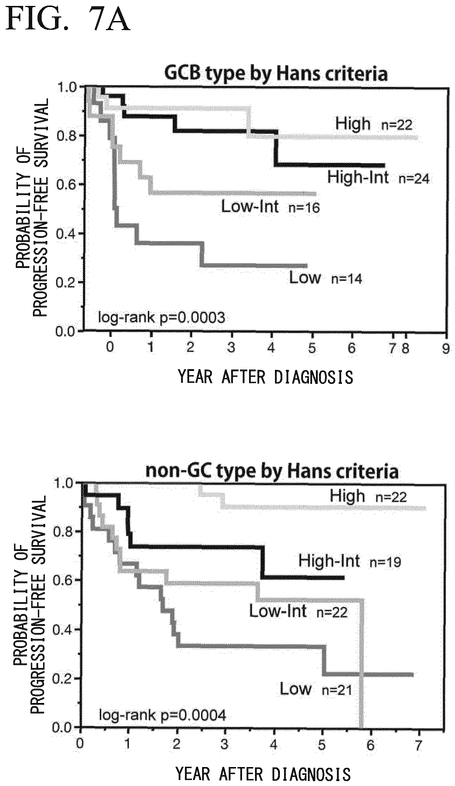

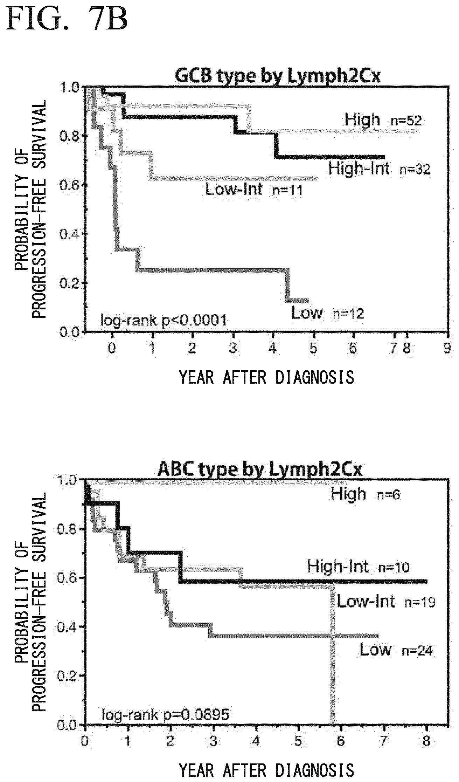

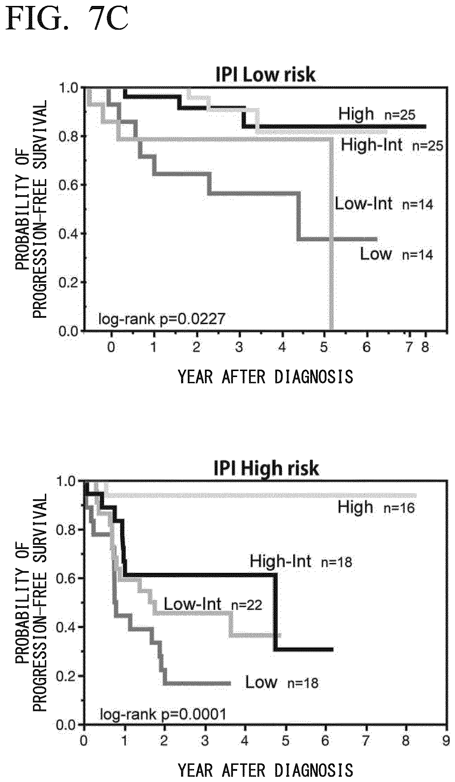

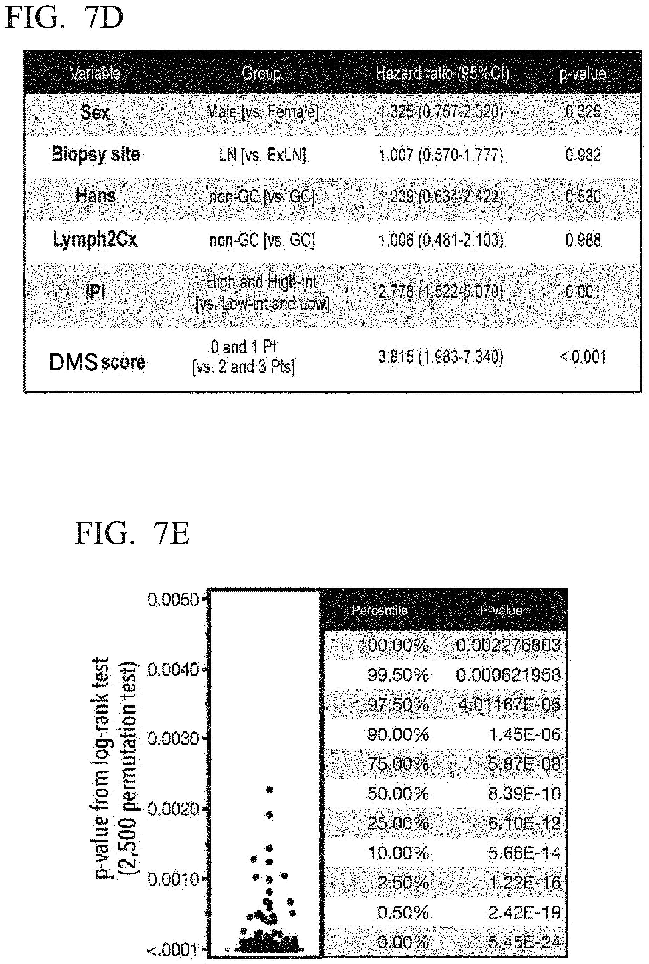

As a result, as shown in FIGS. 7A to 7C, the DMS score could predict prognosis in all subgroups by Hans criteria (GCB and non-GC), all subgroups of Lymph2Cx (GCB and ABC) and IPI classification (high risk (3 to 5) and low risk (0 to 2)). Furthermore, multivariate analysis confirmed that the DMS score was an independent prognostic factor (FIG. 7D). In the permutation test by random resampling technique, 99.50% of p-value from log-rank tests was less than 0.00063. This represents statistical power of the DMS score as a prognostic model (FIG. 7E). Thus, "Tissue-of-Origin" classification defined by enrichment of GC microenvironment signature could predict the clinical outcome of de novo, untreated DLBCL independently with canonical COO classification and IPI.

### (Example 5) Relationship between GC microenvironment signature and gene mutation status in DLBCL

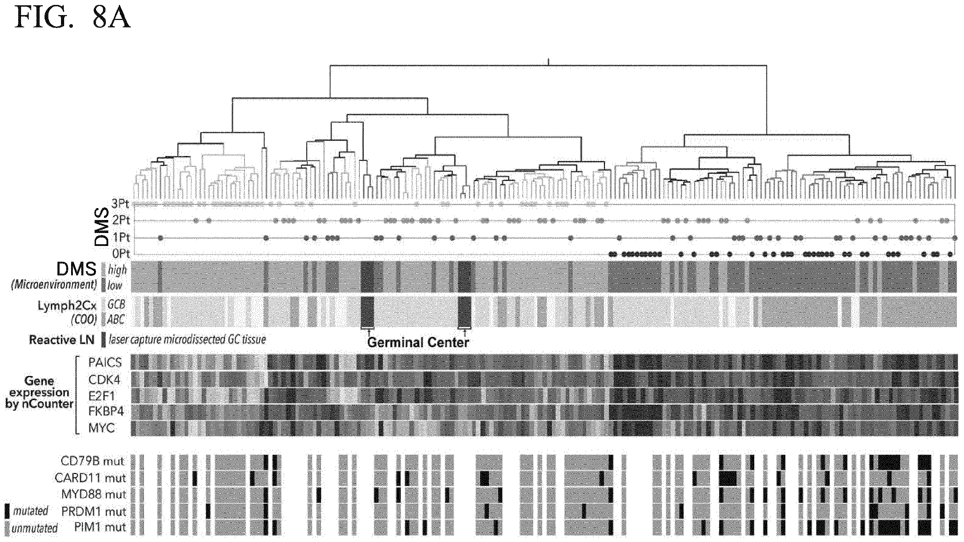

The correlation between the gene expression profile of 447 immune-related genes and the somatic mutation status obtained from target capture sequencing of 106 DLBCL cases using a clinically-validated sequencing platform (OncoPanel, Dana-Farber Cancer Institute) was examined. As a result, unsupervised hierarchical clustering analysis of gene expression profile identified two major clusters, mainly composed of patients with high DMS scores and patients with low DMS scores (FIG. 8A). The gene expression pattern of laser capture microdissected GC tissues from reactive lymph nodes resembled the gene expression pattern of individuals with high DMS scores. This confirmed that the DMS score accurately evaluated the GC microenvironmental tissue.

Also, the patients with the highest DMS score (patients with high GC microenvironment signature) were mutually-exclusive with the individuals with lowest DMS and were characterized by lower expression of genes related to lymphoma activity and cell-cycle such as MYC, CDK4 and E2F1, reflecting their favorable prognosis (FIG. 8A).

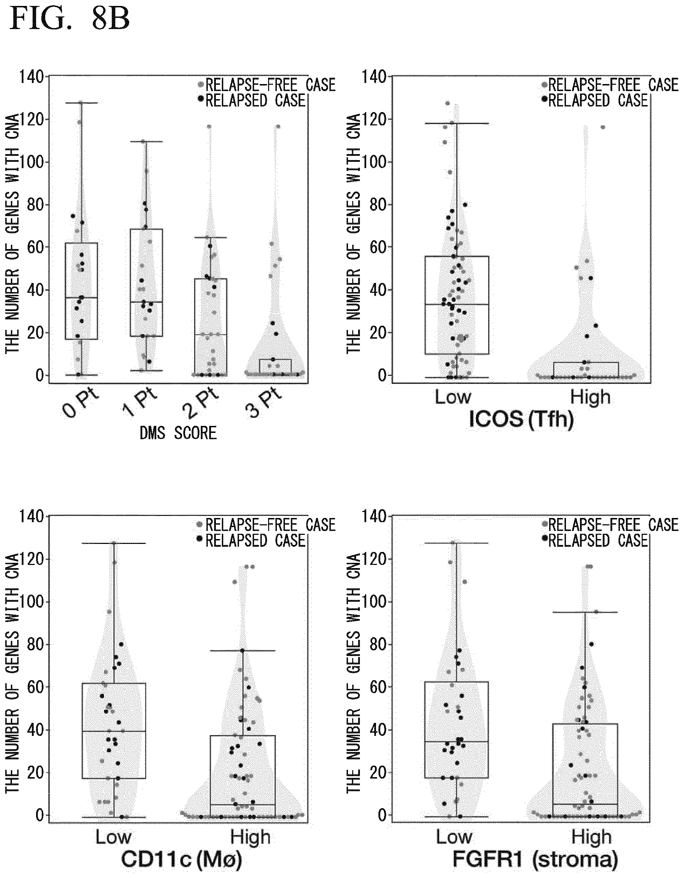

The BCR signaling pathway, which has been repeatedly reported to be enriched in ABC or non-GC subtypes, was significantly concentrated in patients with a low DMS score. This suggests that the microenvironment signature-based subtype correlated with mutation-based subtype. In addition, a high DMS score, especially high-level ICOS expression among the three microenvironment factors, was significantly associated with a decreased number of copy number alteration (CNA) (FIG. 8B). From this fact, it is considered that the GC microenvironment signature in DLBCL correlates with DLBCL subtypes defined by somatic mutations and the number of genes with CNA, presumably affecting pathogenesis or clinical prognosis.

### Industrial Applicability

The present invention provides a method for predicting therapeutic effectiveness of chemotherapy for a DLBCL patient, including: at least one step selected from the group consisting of a step of measuring expression of a marker of a T cell in a sample obtained from the patient, a step of measuring expression of a marker of a macrophage or a dendritic cell in a sample obtained from the patient, and a step of measuring expression of a marker of a stromal cell in a sample obtained from the patient; and a step of predicting prognosis of the treatment, based on the expression of the marker. The present invention contributes to the development of a novel prognostic model of DLBCL and the elucidation of the role of microenvironment components in the pathophysiology of DLBCL.

## Claims

1. A method for predicting therapeutic effectiveness of chemotherapy for a diffuse large B-cell lymphoma patient, comprising:
at least one step selected from the group consisting of a step of measuring expression of a marker of a T cell in a sample obtained from the patient, a step of measuring expression of a marker of a macrophage or a dendritic cell in a sample obtained from the patient, and a step of measuring expression of a marker of a stromal cell in a sample obtained from the patient; and
a step of predicting prognosis of the treatment, based on the expression of the marker.

2. The method according to Claim 1, wherein the step of measuring the expression of the marker is a step of measuring an mRNA expression level of the marker.

3. The method according to Claim 2, wherein the step of predicting the prognosis of the treatment based on the expression of the marker is a step of indicating that the prognosis of the treatment is favorable, when the mRNA expression level of the marker is equal to or more than a cutoff value determined by ROC analysis.

4. The method according to any one of Claims 1 to 3, wherein the chemotherapy is chemotherapy in combination with anti-CD20 antibody.

5. The method according to any one of Claims 1 to 4, wherein the marker of the T cell is at least one marker selected from the group consisting of CCR4, CD2, CD3D, CD3E, CD6, CD7, CD96, FAS, IL2, IL2RB, IL2RG, IL7R, ITK, ITPKB, TNFRSF9, TRAF1, CD40LG, CTLA4, CXCL13, ICOS, IL21, PDCD1, SH2D1A, SLAMF1, and TNFRSF4.

6. The method according to any one of Claims 1 to 5, wherein the T cell is a follicular helper T cell (Tfh cell).

7. The method according to Claim 6, wherein a marker of the Tfh cell is at least one marker selected from the group consisting of CD40LG, CTLA4, CXCL13, ICOS, IL21, PDCD1, SH2D1A, SLAMF 1, and TNFRSF4.

8. The method according to any one of Claims 1 to 7, wherein the marker of the macrophage or the dendritic cell is at least one marker selected from the group consisting of CD80, HLA-DRB1, CD11c (ITGAX), and NOD2.

9. The method according to any one of Claims 1 to 8, wherein the marker of the stromal cell is at least one marker selected from the group consisting of BMP2K, COL8A2, FGFR1, and OBSCN.

10. A kit for predicting therapeutic effectiveness of chemotherapy for a diffuse large B-cell lymphoma patient, comprising:
at least one reagent selected from the group consisting of a reagent for measuring expression of a marker of a T cell, a reagent for measuring expression of a marker of a macrophage or a dendritic cell, and a reagent for measuring expression of a marker of a stromal cell.

11. The kit according to Claim 10, wherein the reagent for measuring the expression of the marker is a reagent for measuring an mRNA expression level of the marker.

12. The kit according to Claim 10 or 11, wherein the chemotherapy is chemotherapy in combination with anti-CD20 antibody.

13. The kit according to any one of Claims 10 to 12, wherein the marker of the T cell is at least one marker selected from the group consisting of CCR4, CD2, CD3D, CD3E, CD6, CD7, CD96, FAS, IL2, IL2RB, IL2RG, IL7R, ITK, ITPKB, TNFRSF9, TRAF1, CD40LG, CTLA4, CXCL13, ICOS, IL21, PDCD1, SH2D1A, SLAMF1, and TNFRSF4.

14. The kit according to any one of Claims 10 to 13, wherein the T cell is a follicular helper T cell (Tfh cell).

15. The kit according to Claim 14, wherein a marker of the Tfh cell is at least one marker selected from the group consisting of CD40LG, CTLA4, CXCL13, ICOS, IL21, PDCD1, SH2D1A, SLAMF 1, and TNFRSF4.

16. The kit according to any one of Claims 10 to 15, wherein the marker of the macrophage or the dendritic cell is at least one marker selected from the group consisting of CD80, HLA-DRB1, CD11c (ITGAX), and NOD2.

17. The kit according to any one of Claims 10 to 16, wherein the marker of the stromal cell is at least one marker selected from the group consisting of BMP2K, COL8A2, FGFR1, and OBSCN.
